# EUROPÄISCHE PATENTSCHRIFT

(11) **EP 3 374 010 B1**
(45) Veröffentlichungstag und Bekanntmachung des Hinweises auf die Patenterteilung: **01.01.2020**
(21) Anmeldenummer: 16788702.5
(22) Anmeldetag: 02.11.2016
(51) Int. Cl.: A61M 15/00

(54) **HANDBETÄTIGBARER INHALATOR**
MANUALLY ACTUATABLE INHALER
INHALATEUR POUVANT ÊTRE ACTIONNÉ MANUELLEMENT

(30) Priorität: 13.11.2015 DE 102015119617
(43) Veröffentlichungstag der Anmeldung: 19.09.2018
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2016/076326
(87) Internationale Veröffentlichungsnummer: WO 2017/080871

(56) Entgegenhaltungen:
- EP-A2- 1 905 470
- DE-A1-102005 033 397
- DE-B3-102005 046 644
- DE-T2- 69 002 353
- JP-A- H1 133 116
- US-A- 5 263 475
- US-A1- 2004 237 276

## Beschreibung

### Gebiet der Technik

Die Erfindung betrifft einen handbetätigbaren Inhalator nach den Merkmalen des Oberbegriffs des Anspruches 1.

### Stand der Technik

Derartige Inhalatoren sind bereits in verschiedener Hinsicht bekannt geworden. Es wird zunächst auf die WO 2006/021546 A1 (vgl. auch US 2007/0289593 A1) verwiesen. Dieser bekannte Inhalator weist ein Schieberteil mit einer Öffnung auf, wobei die Öffnung die Dosierkammer bildet. Das Schieberteil ist zwischen einer in die Vorratskammer eingetauchten Stellung und einer aus der Vorratskammer herausgezogenen Stellung bewegbar. In der aus der Vorratskammer herausgezogenen Stellung befindet sich die Dosierkammer in dem Austragskanal, der unbeweglich ist, und kann durch einen Aussaug-Luftstrom eines Benutzers geleert werden.

Weiter ist auf die DE 199 63 946 A1 zu verweisen (eine modifizierte Ausgestaltung hierzu ist auch durch die US 8 210 170 B2 bekannt geworden), bei welcher der Austragskanal sich in die Vorratskammer erstreckt und axial zwischen einer Spendestellung und einer Füllstellung relativ zu der Dosierkammer, in einer Längsrichtung des Inhalators, bewegbar ist. Die WO 93/ 21980A zeigt einen Inhalator, bei welchem die Dosierkammer in einem Drehteller angeordnet ist und durch Drehen von einer der Vorratskammer zugeordneten Position in eine dem feststehenden Austragskanal zugeordnete Position versetzbar ist.

Im Weiteren ist auch auf die DE 10 2005 033 397 A1 zu verweisen. Bei dem Hieraus bekannten Inhalator ist der Austragskanal feststehend, wobei die Füllung der Dosierkammer im Zuge eines Abschraubens der Überkappe erfolgt. Aus der DE 690 02 353 T2 ist ein Inhalator bekannt, der eine druckbetätigbare Vorratskammer aufweist mit unter Druck stehender Substanz. Bei der Druckbetätigung wird die Substanz sprühend ausgegeben. Aus der US 5 263 475 A ist ein Inhalator bekannt, der gleichfalls über eine Vorratskammer verfügt, welche auf Druckbetätigung hin Substanz aussprüht. Hierbei ist ein als Ventil wirkendes Teil in die Vorratskammer eingefahren. Weiter ist aus der US 2004/0237276 A1 ein Inhalator bekannt, bei welchem der Austragskanal drehbar ist. Die Dosierkammer wird beim Stillstand des Austragskanals durch Schwerkraft gefüllt.

### Zusammenfassung der Erfindung

Ausgehend von dem dargelegten Stand der Technik beschäftigt sich die Erfindung mit der Aufgabenstellung, einen Inhalator zur Ausbringung einer pulverförmigen Substanz anzugeben, bei welchem eine vorteilhafte Füllbarkeit der Dosierkammer erreicht ist.

Diese Aufgabe ist beim Gegenstand des Anspruches 1 gelöst, wobei darauf abgestellt ist, dass der Austragskanal um eine Drehachse drehbar ist und dass die Dosierkammer im Zug einer Drehung des Austragskanals füllbar ist-, dass die Dosierkammer auf ihrer dem Austragskanal gegenüber liegenden Seite eine durch einen Stopfen verschließbare Öffnung aufweist, und dass die Dosierkammer von dem Stopfen abhebbar ist.

Weiter ist auch eine günstige Verwirbelung in dem Austragskanal zu erreichen und kann die Verschlusskappe günstig auf den Austragskanal einwirken.

Die Drehbarkeit des Austragskanals ermöglicht hinsichtlich einer Befüllung der Dosierkammer unterschiedliche Ausgestaltungen. Die Dosierkammer kann zur Befüllung durch die Drehung freigegeben werden, beispielsweise damit aus der Dosierkammer Substanz in die freigegebene Dosierkammer eintreten kann. Die Dosierkammer kann aber auch durch die Drehung des Austragskanals durch an diesem befindliche Mittel zwangsgefüllt werden. Auch eine Kombination der Maßnahmen ist möglich. Die Vorratskammer kann zusammen mit dem Austragskanal für eine Versetzung der Dosierkammer von einem geschlossenen in einen geöffneten Zustand und umgekehrt relativ zu dem Mundstück bewegbar sein.

Betreffend die Verwirbelung können in Durchströmungsrichtung nachfolgend die ersten Teilströme in einem Unterbrechungsbereich in zweite, danach auch nebeneinander geführte Teilströme aufgeteilt werden, wobei ein zweiter Teilstrom auf einer Zusammenführung von Anteilen der beiden ersten Teilströme beruht.

Hinsichtlich einer Zusammenwirkung des Mundstücks mit dem Austragskanal kann vorgesehen sein, dass der Austragskanal relativ zu dem Mundstück aus einer ersten Stellung, in der das Ende des Austragskanals der Mündungsebene näher ist, in eine zweite Stellung, in der das Ende des Austragskanals der Mündungsebene ferner ist, und umgekehrt, bewegbar ist und dass an der Verschlusskappe ein Einwirkungsabschnitt vorgesehen ist, zur Bewirkung jedenfalls der Bewegung des Austragskanals aus der ersten in die zweite Stellung.

Der handbetätigbare Inhalator als solcher ist bevorzugt langgestreckt und im Wesentlichen zylindrisch ausgebildet. Wesentliche Bestandteile, die bei üblicher Handhabung von einem oberen bis zum unteren Ende gegeben sind, können zunächst eine obere Verschlusskappe sein, weiter ein Mundstück, das nach Abnahme der Verschlusskappe offen liegen kann. Darüber hinaus der genannte Austragskanal, der prinzipiell aber auch das Mundstück bereits umfassen kann. Daran kann sich dann die Vorratskammer mit darin hineinragendem Austragskanal anschließen und zugeordnet zu der Vorratskammer die Dosierkammer, die mit Substanz aus der Vorratskammer jeweils befüllbar ist.

Im Weiteren können insbesondere noch ein Reservoir für Entfeuchtungsmittel und/oder Verwirbelungsmittel betreffend den austretenden, mit Substanz beladenen Luftstrom und/oder ein oder mehrere Führungskanäle für von außen angesaugte oder eingebrachte Luft und/oder ein Zählwerk vorgesehen sein.

Dadurch, dass der Austragskanal um eine Drehachse drehbar und dass die Dosierkammer im Zuge einer Drehung des Austragskanals füllbar ist, ist die Füllung der Dosierkammer an die Drehung des Austragskanals gekoppelt. In konkreterer Hinsicht bewirkt die Drehung des Austragskanals zu einem wesentlichen Teil die Füllung der Dosierkammer. Da mit der Drehung des Austragskanals, der in den Vorratsraum hineinragt, auch eine Beeinflussung der Substanz in dem Vorratsraum verbunden ist, wird zugleich diese Beeinflussung der Substanz in dem Vorratsraum und die Befüllung der Dosierkammer erreicht.

Im Hinblick auf die Ausgestaltung, dass die Vorratskammer zusammen mit dem Austragskanal relativ zu dem Mundstück bewegbar ist, ist eine Bewegbarkeit der Baueinheit von Austragskanal und Vorratskammer erreicht. Sie kann beispielsweise zu einer günstigen Öffnung und Schließung einer Dosierkammer genutzt sein.

Hinsichtlich der Verwirbelung lässt die Aufteilung in erste Teilströme und die Zusammenführung in zweite Teilströme eine nochmals günstigere Aufschließung des letztlich zu inhalierenden pharmazeutischen Anteils der Substanz erreichen. Es kommt zu Kollisionen zwischen Teilen der pulverförmigen Substanz selbst. Die Loslösung einer Wirksubstanz von dem Trägermaterial kann vorteilhaft erreicht werden.

Hinsichtlich der Beeinflussung des Austragskanals durch die Verschlusskappe kann durch Aufbringen oder Abziehen der Verschlusskappe günstig eine Bewegung des Austragskanals bewirkt werden. Insbesondere in einer Aufsteck- oder Aufdrehrichtung der Verschlusskappe auf das Mundstück. Wenn im Zuge einer Nutzung des Inhalators der Austragskanal näher an die Mündungsebene herangeführt worden ist, kann durch Aufsetzen der Verschlusskappe sicher erreicht werden, dass der Austragskanal in eine diesbezügliche Ausgangsstellung zurückversetzt wird.

In weiterer Ausgestaltung ist bevorzugt, dass die Drehachse des Austragskanals zumindest teilweise zusammenfällt mit einer Transportrichtung von durch den Austragskanal transportierter Substanz. Bevorzugt ist die Drehachse auch im Wesentlichen eine geometrische Mittel-Längsachse des bevorzugt im Wesentlichen zylindrisch gestalteten Inhalators insgesamt und/oder eine geometrische Mittellängsachse eines Teils des Austragskanals.

Weiter bevorzugt ist die Dosierkammer zu der Drehachse des Austragskanals exzentrisch angeordnet. Hierdurch lässt sich in konstruktiv weiterhin günstiger Weise die genannte Befüllung, aber auch eine Entleerung, insbesondere eine Freigabe zur Entleerung durch Drehung des Ausgangskanals, der Dosierkammer erreichen.

Der Austragskanal durchsetzt die Dosierkammer bevorzugt derart, dass er über jedenfalls einen Teil seiner die Dosierkammer durchsetzenden Länge auf seinem gesamten Umfang quer zu einer Durchsetzungsrichtung von der Substanz umgeben sein kann. Somit ist sichergestellt, dass zwangsläufig die Substanz durch den sich drehenden Austragskanal beeinflusst, gleichsam gerührt wird. Dies ist insbesondere auch vorteilhaft im Zusammenhang mit der genannten exzentrischen Anordnung der Drehachse.

Die Dosierkammer ist weiter bevorzugt von dem Austragskanal gesondert ausgebildet. Hierbei kann die Dosierkammer ein Teilbereich der Vorratskammer sein, der unmittelbar von dem sonstigen Bereich der Vorratskammer aus zugänglich ist, aber außerhalb des von dem drehenden Austragskanal bei einer vollständigen Drehung insgesamt eingenommenen Raumes ausgebildet ist. Dadurch, dass die Dosierkammer von dem Austragskanal gesondert ausgebildet ist, ist keine Befüllung in den Austragskanal hinein vorgesehen. Andererseits kann die Dosierkammer, in der Spendestellung, sich in unmittelbarem räumlichen Anschluss an den Austragskanal oder an eine zu diesem führende Öffnung befinden, die dann beim Austragen von ausgetragener Substanz durchströmt ist.

Insbesondere ist auch bevorzugt, dass die Dosierkammer bodenseitig der Vorratskammer angeordnet ist. So kann bei üblicher Handhabung allein schon durch Schwerkraft bei einem Freigeben der Dosierkammer Substanz zum Nachfüllen in die Dosierkammer gelangen. Dies bedeutet bevorzugt aber auch, dass der Austragskanal sich durch die gesamte Vorratskammer hindurch erstreckt. Diese Erstreckung ist betrachtet bezogen auf eine Ausrichtung des Inhalators mit dem Mundstück oben und dem Boden der Vorratskammer unten, so dass in dieser Betrachtung der Austragskanal die Vorratskammer in Vertikalrichtung vollständig oder nahezu vollständig durchsetzt.

Der Austragskanal kann einen Fuß aufweisen, der im Zuge einer Drehung des Austragskanals die Dosierkammer zur Füllung freigibt. In gleicher Weise kann dieser Fuß dann auch bei weiterer Drehung die Dosierkammer wieder abdecken. Der Fuß kann insbesondere über den bei der angesprochenen Betrachtung in Vertikalrichtung sich oberhalb des Fußes erstreckenden Bereich des Austragskanals bezogen auf die Drehachse oder eine Längserstreckung des Austragskanals radial über diesen hinausragen. Der Fuß kann auch zugleich eine Strömungskammer bilden, durch welche bei Überdeckung zur Dosierkammer hieraus austretende Substanz in den sich anschließenden Bereich des Austragskanals geleitet werden kann.

Eine Unterseite des Austragskanals, die ggf. durch den angesprochenen Fuß gebildet ist, ist bevorzugt ebenengleich zu einem Boden der Vorratskammer außerhalb der Dosierkammer verlaufend gestaltet. Somit kann mit der Unterseite des Austragskanals der Boden der Dosierkammer bei Drehung des Austragskanals überfahren werden. In dieser Beziehung ist bevorzugt die Dosierkammer unterhalb des Bodens bzw. von dem Boden der Dosierkammer nach unten sich erstreckend ausgebildet. Wenn sich die Unterseite des Austragskanals oberhalb der Dosierkammer, praktisch diese verschließend, befindet, bildet sie entsprechend gleichsam einen Deckel. Es handelt sich aber nicht notwendig um einen Deckel im üblichen Sinne, da der genannte Strömungsweg zum Austragen sich hierin bereits ausbilden kann und hierdurch gegeben sein kann. Durch das Überfahren der gefüllten Dosierkammer mit dem Fuß oder der Unterseite des Austragskanals kann auch einer Überfüllung der Dosierkammer entgegengewirkt werden. Es kann eine vorteilhaft gleichmäßige Befüllung erreicht werden.

Der Austragskanal weist insbesondere bevorzugt hinsichtlich einer Erstreckung in die Vorratskammer einen abgewinkelten Verlauf auf. Die Erstreckung in die Vorratskammer ist im Hinblick auf die angesprochene Betrachtung bei Ausrichtung des Inhalators mit einem Mundstück oben und dem Boden der Dosierkammer unten, die Dosierkammer im Wesentlichen von oben nach unten, also vertikal, durchsetzend. Der abgewinkelte Verlauf, zusammen mit der genannten Drehbarkeit, kann entsprechend dazu führen, dass ein insbesondere unterer Bereich des Austragskanals einen relativ großen Kreis bezogen auf den Boden der Dosierkammer bei einer Drehung beschreibt, gegenüber einem kleinen bzw. sich nur um die eigene (geometrische) Längsachse drehenden Bereich weiter oberhalb. Diese Abwinkelung erbringt ersichtlich auch einen Rühreffekt bezüglich der Substanz in der Vorratskammer. Zudem kann sie günstig zur Überdeckung und Freigabe der gesamten Dosierkammer durch die Verdrehung um die Drehachse beitragen.

Die Dosierkammer ist auf ihrer dem Austragskanal gegenüberliegenden Seite bzw. auf einer in Strömungsrichtung beim Austragen von Substanz vorgelagerten Seite mit einer durch einen Stopfen verschließbaren Öffnung versehen. Dieser Stopfen kann insbesondere zur Freigabe eines Austrags-Luftstroms genutzt sein. Wenn der Stopfen aus der Öffnung entfernt ist, kann der Austrags-Luftstrom hindurchtreten.

Die Dosierkammer ist von dem Stopfen abhebbar. Einerseits kann hierzu der Stopfen relativ zu einer feststehenden Dosierkammer beweglich vorgesehen sein. Da die Dosierkammer ein Teil der Vorratskammer ist, kann andererseits und bevorzugt auch vorgesehen sein, dass die Dosierkammer zusammen mit der Vorratskammer im Übrigen derart bewegbar ist, dass sich die gewünschte Abhebung von dem Stopfen ergibt. Bei bevorzugter Ausgestaltung entspricht dies einer Bewegung der Dosierkammer und/oder der Vorratskammer in Vertikalrichtung bzw. in Ausrichtung der Drehachse des Austragskanals. In weiter bevorzugter Ausgestaltung ist die Vorratskammer zusammen mit dem Austragskanal bewegbar, auf ein Mundstück hin oder von dem Mundstück weg. Insbesondere im Zusammenhang mit dem angesprochenen Stopfen kann durch die Bewegung die Dosierkammer unterseitig freigegeben werden, so dass von einem Nutzer eingesaugte Luft bodenseitig der Dosierkammer, welcher Boden dann durch die darin enthaltene Substanz gleichsam gebildet ist, auftrifft und den Inhalt der Dosierkammer in den dann zu der Dosierkammer ausgerichtet angeordneten Austragskanal einsaugt. Die Dosierkammer wird als Teil der Vorratskammer von dem feststehenden Stopfen abgehoben. Bei entgegengesetzter Bewegung wird die Dosierkammer hierdurch, durch Aufsetzen auf den Stopfen, unterseitig geschlossen.

Insbesondere kann also die Dosierkammer zusammen mit dem Austragskanal und/oder nur dem Boden der Vorratskammer in der genannten Weise bewegbar sein.

Der Austragskanal kann insbesondere als Saugkanal ausgebildet sein. Durch Ansaugen von Luft durch einen Benutzer ergibt sich bei gefüllter Dosierkammer zunächst das Abheben der Dosierkammer von dem Stopfen (oder umgekehrt). Damit ist dann ein Strömungsweg durch die Dosierkammer hindurch in den Austragskanal und von dort durch das Mundstück in einen Mund eines Benutzers geöffnet.

Das Abziehen der Dosierkammer von dem Stopfen oder das Herausziehen des Stopfens aus einem betreffenden Bodenbereich der Dosierkammer kann mit einer bewussten erforderlichen Kraftaufwendung versehen sein. Hierdurch wird der Benutzer gezwungen, eine gewisse Unterdruckschwelle beim Ansaugen zu überschreiten. Sobald der Stopfen abgehoben hat, ergibt sich dann zwangsweise auch durch die dann durchströmende Luft eine gewünschte rasche und vollständige Entleerung der Dosierkammer durch den Austragskanal.

Hinsichtlich eines Ablaufes beim Durchführen einer Inhalation durch einen Nutzer ist in einer Ausgangsstellung, wenn die Verschlusskappe noch vollständig aufgesetzt ist, der Austragskanal, insbesondere hinsichtlich seines Fußes und weiter insbesondere hinsichtlich des in dem Fuß gegebenen freien Strömungsquerschnittes, nicht in Überdeckung zu der Dosierkammer. Im Zuge einer Abnahme, insbesondere eines Abschraubens, der Verschlusskappe wird vielmehr der Austragskanal erst in die für das Inhalieren erforderliche Überdeckung zu der Dosierkammer bewegt, insbesondere gedreht. Bevorzugt vollzieht der Austragskanal hierbei eine Drehung um eine diesbezügliche Drehachse, die weniger als 360° beträgt. Beispielsweise im Bereich von 270° - 330°, weiter bevorzugt bei 300°. Diese zwangsläufig gegebene Drehung im Zuge der Überführung des Inhalators in eine Inhalations-Bereitschaftsstellung sorgt zwingend dafür, dass eine Beeinflussung der Substanz in dem Vorratsraum durch den drehenden Austragskanal jeweils vor einer Inhalation erreicht wird. Eventuelle durch Anhaften in der Substanz gegebene Brücken oder dergleichen können aufgebrochen werden. Zudem kann diese Drehung zu einer schaufelnden Befüllung der Dosierkammer, insbesondere durch den Fuß des Austragskanals, genutzt sein.

### Kurze Beschreibung der Zeichnungen

Nachstehend ist die Erfindung des Weiteren anhand der beigefügten Zeichnung erläutert, die jedoch lediglich ein Ausführungsbeispiel darstellt. Hierbei zeigt:
- Fig. 1: eine Außenansicht des Inhalators auf der Seite, auf welcher eine Zählwerk-Anzeige ausgebildet ist;
- Fig. 2: eine Explosionsansicht des Inhalators;

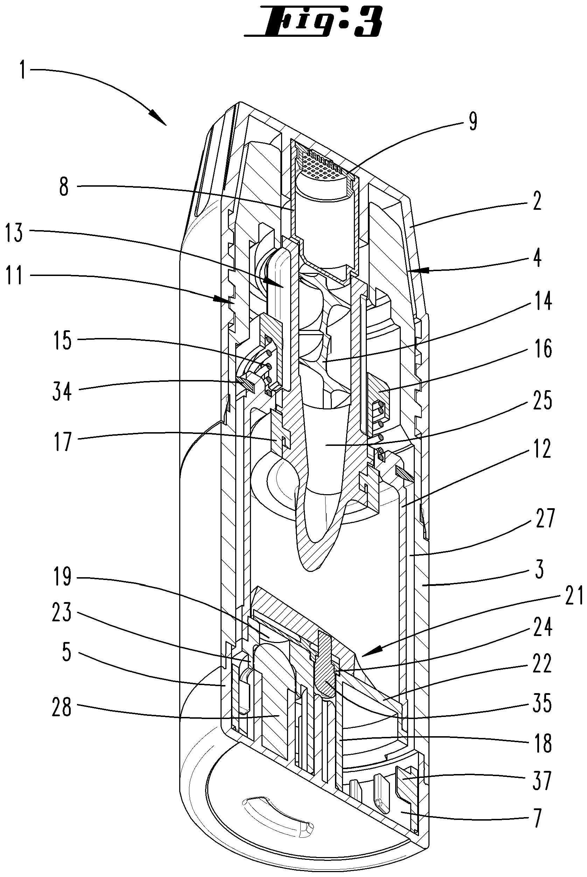

- Fig. 3: einen perspektivischen Längsschnitt durch den Inhalator;
- Fig. 4: einen Längsschnitt durch den Inhalator im Zuge des Abschraubens der Abdeckkappe;
- Fig. 5: einen weiteren Längsschnitt durch den Inhalator mit gefüllter Dosierkammer;
- Fig. 6: einen Längsschnitt gemäß Fig. 5, bei über die Dosierkammer gefahrenem Austragskanal und bei vom Stopfen abgehobener Dosierkammer;
- Fig. 7: eine Außenansicht des Austragskanals;
- Fig. 8: einen Querschnitt durch den Gegenstand gemäß Fig. 7, geschnitten entlang der Linie VIII-VIII;
- Fig. 9: eine Außenansicht des Mundstücks mit Rumpfteil;
- Fig. 10: eine Ansicht des Inhalators von oben, bei abgenommener Verschlusskappe;
- Fig. 11: eine Unterseite der Verschlusskappe; und
- Fig. 12: eine gesonderte perspektivische Darstellung des Verwirbelungsmittels.

### Beschreibung der Ausführungsformen

Dargestellt und beschrieben ist ein Inhalator 1 zur Ausbringung einer pulverförmigen Substanz. Bei der pulverförmigen Substanz handelt es sich insbesondere um eine pharmazeutische Substanz. Es kann ein Wirkmittel in dem Pulver enthalten sein, das von einem Benutzer durch Einatmen aus dem Inhalator entnommen werden kann.

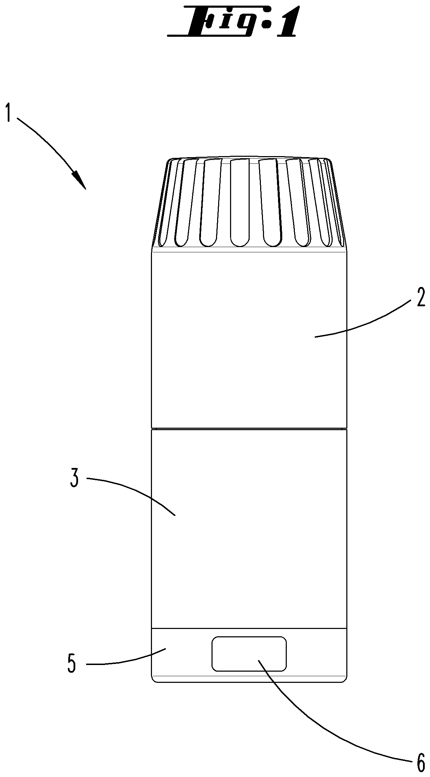

In der Ansicht der Fig. 1 ist eine obere Verschlusskappe 2 zu erkennen, darunter ein Rumpfteil 3, das wie weiter noch erläutert, mit einem Mundstück 4 einheitlich gebildet sein kann. Darüber hinaus ein Bodenteil 5, das in der Ansicht der Fig. 1 ein Fenster 6 aufweist. Durch das Fenster 6 kann ein Zählring 7 eines in dem Bodenteil bevorzugt vorgesehenen Zählwerks sichtbar sein.

Der Inhalator 1 weist ersichtlich eine im Wesentlichen zylindrische Gestaltung auf. Beim Ausführungsbeispiel und bevorzugt ist ein oberer Teil der Verschlusskappe 2 sich zu dem diesbezüglichen Ende hin konisch verjüngend gestaltet.

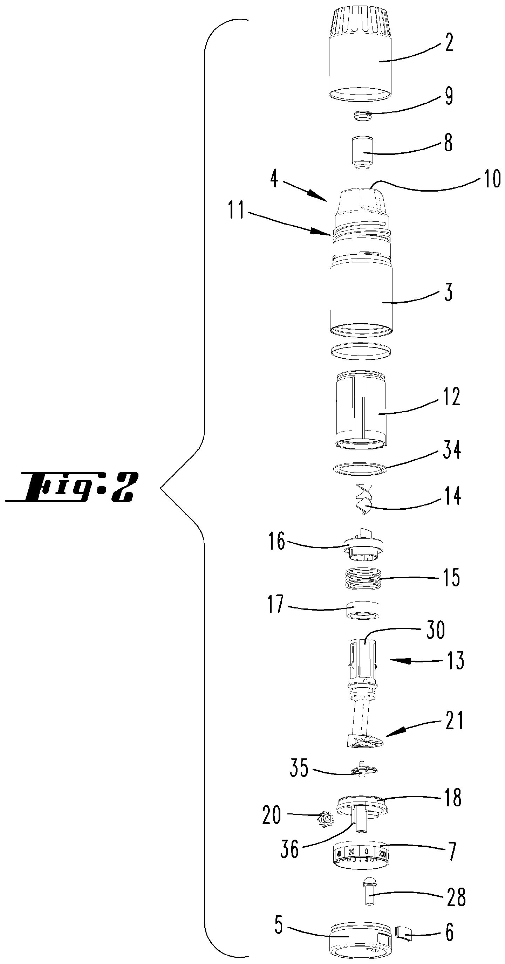

Die Teile des Inhalators 1 sind im Weiteren aus der Explosionsdarstellung der Fig. 2 ersichtlich. Innerhalb der Verschlusskappe 2 kann ein Reservoir 8 angeordnet sein. In dem Reservoir 8 kann Entfeuchtungsmittel enthalten sein. Das Reservoir 8 kann über ein Verbindungsteil 9 im Inneren der Kappe 2 befestigt sein, beispielsweise rastbefestigt.

Die Kappe 2 überfängt im zusammengesetzten Zustand das Rumpfteil 3, das beim Ausführungsbeispiel materialeinheitlich einteilig mit einem Mundstück 4 ausgebildet ist.

Das Mundstück 4 bzw. das Rumpfteil 3 weist unterhalb des von einem Benutzer nutzbaren Mündungsabschnittes 10 ein Außengewinde 11 auf, das mit einem Innengewinde in der Verschlusskappe 2 zusammenwirken kann. Im Zuge des Abschraubens können weiter ein oder mehrere Mitnahmeelemente 29 an der Verschlusskappe 2 mit entsprechenden Mitnahmeausformungen 30 an einem Austragskanal 13 zusammenwirken, so dass sich eine Drehung des Austragskanals 13 im Zuge eines Abschraubens der Verschlusskappe 2 ergibt.

Innerhalb des Rumpfteils 3 kann sich die Vorratskammer 12 erstrecken, bevorzugt als gesonderte Kammer aufgenommen sein, die von dem Austragskanal 13 durchsetzt ist. In dem Austragskanal 13, bevorzugt zugeordnet dem Mundstück 4, kann ein Verwirbelungsmittel 14 angeordnet sein. Die Vorratskammer 12 kann mittels einer Feder 15 gegen ein Widerlager 16 abgestützt sein. Das Widerlager 16 kann bei aufgesetzter Kappe beispielsweise durch ein Mitnahmeelement 29 druckbeaufschlagt sein, derart, dass vermittels der Feder 15 auf die Vorratskammer 12 eingewirkt wird. Das Widerlager 16 kann axial verschiebbar, gegebenenfalls auch drehgehindert, an dem Austragskanal 13 geführt sein. Der Austragskanal 13 kann jedenfalls in der Vorratskammer 12 relativ zu der Vorratskammer 12 drehbar angeordnet sein.

Es kann weiter ein Vorratskammer-Bodenteil 18 vorgesehen sein, in dem auch die Dosierkammer 19 ausgebildet sein kann. Unterhalb des Vorratskammer-Bodenteils 18 können der Zählring 7 und ein dieses beaufschlagendes Antriebsrad 20 angeordnet sein.

In dem Bodenteil 5 kann ein Stopfen 28 gehaltert sein, welcher die Dosierkammer 19 verschließt bzw. freigibt.

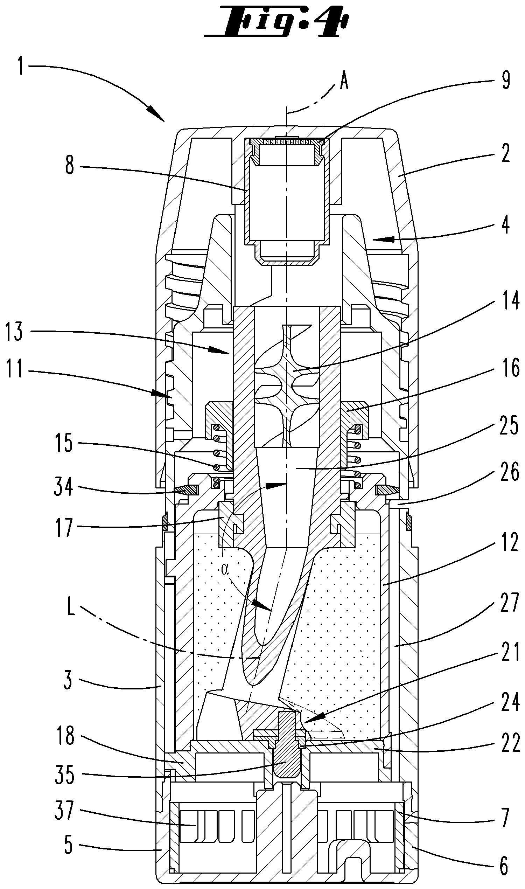

Der Austragskanal 13, wie sich etwa auch aus einem Vergleich der Figuren 4 bis 6 ergibt, ist um eine Drehachse A in dem Inhalator 1 drehbar angeordnet. Die Drehachse A fällt bevorzugt und beim Ausführungsbeispiel mit einer geometrischen Achse des Inhalators 1 in Bezug auf seine zylindrische Gestaltung zusammen.

Die Bewegbarkeit des Austragskanals 13 um die Drehachse A lässt erreichen, dass die Dosierkammer 19 im Zuge einer Drehung des Austragskanals 13 füllbar ist.

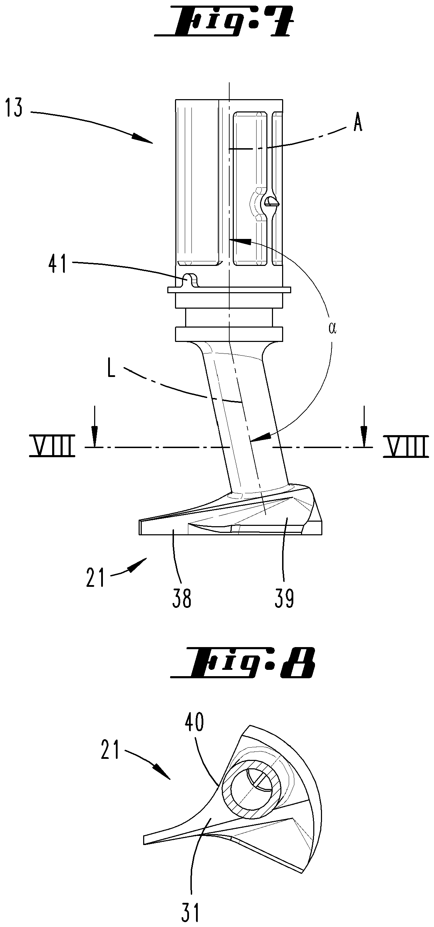

Der Austragskanal 13 weist bevorzugt und beim Ausführungsbeispiel einen Fuß 21 auf. Der Fuß 21 überstreicht bei einer Drehung des Austragskanals 13 um die Drehachse A den Vorratskammer-Boden mit wechselnder, insgesamt einer Kreis-Ringfläche entsprechenden Überdeckung. Der Fuß ist in weiterer Einzelheit insbesondere aus den Figuren 7 und 8 ersichtlich. Der Fuß weist eine Ausformung 31 auf, die abweichend von einer Kreisform ist (bei Ansicht in Richtung der Drehachse A) in Bezug auf den Vorratskammer-Boden 22. So kann im Zuge einer Drehung jeweils ein Teil des Vorratskammer-Bodens freigelegt sein und ein anderer von dem Fuß 21 überdeckt sein.

Die Dosierkammer 19 ist in dem Vorratskammer-Boden 22 ausgebildet. Beim Ausführungsbeispiel und bevorzugt handelt es sich um eine Kammer, die sich ausgehend von einer Oberfläche des Vorratskammer-Bodens 22 nach unten erstreckt. Es kann eine umlaufende, beispielsweise zylindrische, Kammerwand 23 ausgebildet sein.

Die Dosierkammer 19 ist bevorzugt als freie Durchgangsöffnung gebildet. Sie kann in Abhängigkeit einer bestimmten Drehstellung des Austragskanals 13 oberseitig durch den Austragskanal 13 oder, soweit ausgebildet, deren Fuß 21, abgedeckt sein. Unterseitig kann sie durch den Stopfen 28 verschlossen sein.

Der Stopfen 28 ist zur Entleerung der Dosierkammer 19 aus der Dosierkammer herausfahrbar vorgesehen.

Der Stopfen 28 besteht bevorzugt aus einem weichen, beispielsweise elastomeren Teil. Es kann sich auch um einen Gummiwerkstoff handeln. Es kann sich auch um ein thermoplastisches Elastomer handeln.

Alternativ kann auch die Kammwandung 23 aus einem elastischen Werkstoff bestehen und der Stopfen 20 aus einem Hartwerkstoff, wie etwa Hartkunststoff.

Die Zusammenwirkung des Stopfens 20 mit der Kammerwand 23 ist bevorzugt so eingestellt, dass eine gewisse Schwellkraft überwunden werden muss, um den Stopfen 20 aus der Dosierkammer 19 zur Öffnung der Dosierkammer 19 herauszuziehen.

Diese Schwellkraft ist an den von einem menschlichen Benutzer erzeugbaren Unterdruck im Zuge der Durchführung eines Inhalier-Vorganges angepasst.

Der Austragskanal 13 kann über einen im unteren Bereich der Vorratskammer 12 vorgesehenen Lagervorsprung 24 unterseitig gelagert sein. Der Lagervorsprung 24 kann in einer entsprechenden Lagerausnehmung des Vorratskammer-Bodenteils 22, bevorzugt zentral bezüglich der Drehachse A, aufgenommen sein. Er ragt ersichtlich oberseitig mit einem Zapfenabschnitt in einen unteren Bereich des Austragskanals 13 oder speziell beim Ausführungsbeispiel in den Fuß 21. Im Bereich des Lagervorsprungs 24, auf einem gewissen Radius zu einer Drehachse des Lagervorsprungs 24, kann sich auch ein Bereich ergeben, in welchem der Vorratskammer-Boden 22 ständig abgedeckt ist, unabhängig von der Drehstellung des Austragskanals 13.

Wie sich weiter auch aus den Figuren 7 und 8 ergibt, ist der Fuß 21 an seinen Randkanten schaufelartig gebildet. Im Zuge einer Drehung des Austragskanals 13 bzw. des Fußes 21 kann so gleichsam eine Beschaufelung der Dosierkammer 19, zu deren Befüllung erfolgen.

Der Austragskanal 13 ist weiter bevorzugt und beim Ausführungsbeispiel mit einem abgeknickten Verlauf versehen. Während in seinem oberen Bereich, zugeordnet einem oberen Ende der Vorratskammer 12 und bis in Erstreckung zu dem Mundstück 4 eine im Wesentlichen rotationssymmetrische Erstreckung zu der Drehachse A gebildet ist, ist in dem unteren Bereich, beim Ausführungsbeispiel und bevorzugt allein in der Vorratskammer 12, eine Abknickung vorgesehen. Eine Längsachse L des abgeknickten Bereichs schließt mit der Drehachse A bevorzugt einen stumpfen Winkel α von 100° -175° ein. Beim Ausführungsbeispiel und bevorzugt sind es 155° - 175°, weiter bevorzugt ca.168°: Weiter bevorzugt setzt sich der Austragskanal aus wenigen, darüber hinaus bevorzugt nur zwei, für sich jeweils gerade verlaufenden Abschnitten zusammen.

Der Austragskanal 13 weist eine innere Höhlung 25 auf, durch welche im Zuge eines Inhalierens mit Substanz befrachtete Ansaugluft von dem Vorratskammer-Boden zu dem Mundstück 4 transportiert wird.

Ersichtlich und bevorzugt ist diese Höhlung 25 im Bereich der Längsachse L mit einem kleineren Durchmesser gebildet und erweitert sich sodann zu einem größeren Durchmesser.

Im Bereich des größeren Durchmessers und vorgeschaltet zu dem Mundstück 4 in Ausströmrichtung ist das Verwirbelungselement 14 angeordnet. In dem Rumpfteil 3 sind, bevorzugt im Hinblick auf die Drehachse A im Bereich der Dichtung 17, ein oder mehrere Durchlassöffnungen 26 ausgebildet. Bei abgeschraubter Verschlusskappe 2 kann durch diese Durchlassöffnungen 26 durch den Benutzer Luft von außen angesaugt werden, welche über einen beispielsweise als Ringraum ausgestalteten Luftkanal 27 unterhalb des Vorratskammer-Bodens 22 strömt und weiter durch die Dosierkammer 19, unter Austreiben der darin dann enthaltenen Substanz, und beladen mit der Substanz in den Höhlung 25 des Austragskanals 13.

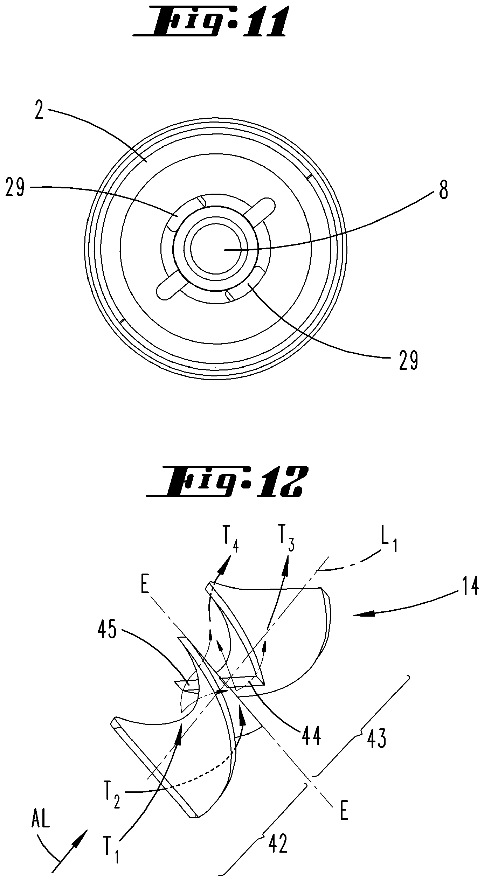

Das Verwirbelungsmittel 14 ist in Figur 12 in weiterer Einzelheit dargestellt. Es besteht aus in axialer Richtung hintereinander angeordneten Teilbereichen 42 und 43. Jeder Teilbereich 42 und 43 entspricht bevorzugt für sich einer üblichen wendelförmigen Verwirbelungseinrichtung. Zwischen den Teilbereichen 42 und 43 sind diese Teilbereiche jedoch in einer Ebene E-E, die senkrecht zu einer Längsachse L₁ des Verwirbelungsmittels 14 verläuft, gleichsam aus einem diese Teilbereiche gedanklich enthaltenden kontinuierlichen Wendelteil getrennt und dann winkelmäßig versetzt, und zwar bevorzugt um einen Drehwinkel von 90°, wieder zusammengesetzt. Somit ergibt sich für in den ersten, unteren bzw. in Strömungsrichtung ersten Teilbereich 42 einströmende, mit Substanz befrachtete Ansaugluft AL, dass diesbezügliche erste Teilströme T₁ und T₂ auf diesbezügliche Teilungskanten 44, 45 des zweiten Teilbereichs 43 treffen, welche die erste Teilströme T₁, T₂ in zweite Teilströme T₃, T₄ aufteilen, wobei diese zweiten Teilströme T₃, T₃ wiederum auf einer Zusammenführung von Teilen der ersten Teilströme T₁, T₂ beruhen. Das Verwirbelungsmittel 14 ist bevorzugt auch so gestaltet, dass jeder Teilstrom T₁ bis T₄ so gelenkt wird, dass er etwa eine Viertelumdrehung oder mehr um die Längsachse L₁ vollzieht.

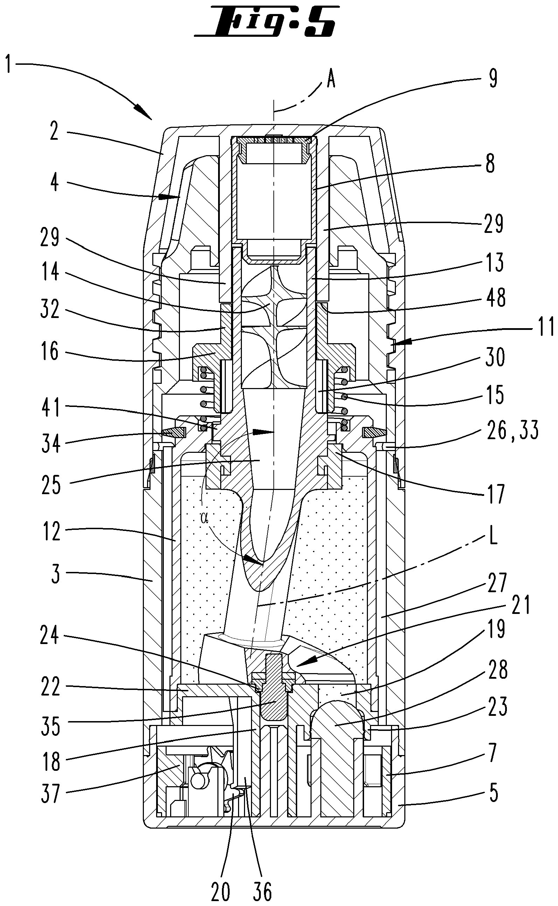

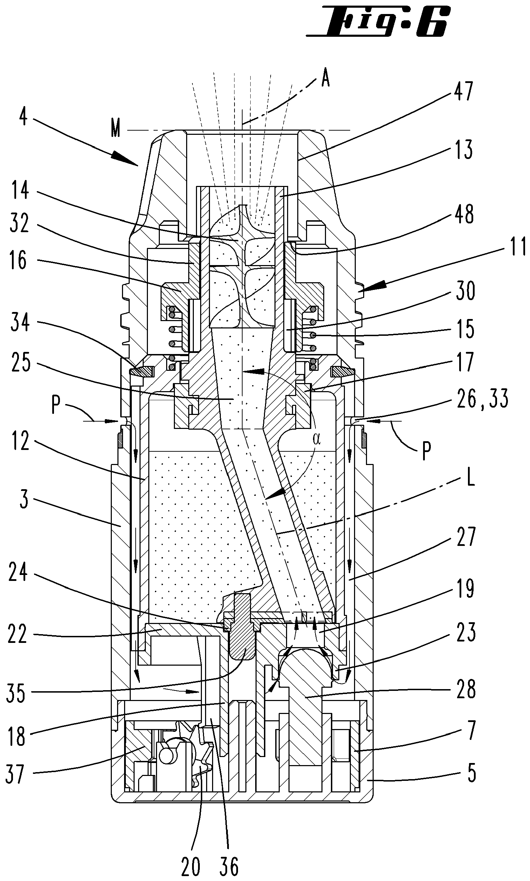

Wie insbesondere aus einem Vergleich der Figuren 5 und 6 ersichtlich, wird im Zuge eines Abschraubens durch Herausfahren der Mitnahmeelemente 29 aus den Mitnahmeausformungen 30, solange diese aber noch in Überdeckung sind, eine Drehung des Austragskanals 13 bewirkt. Diese Drehung kann bevorzugt bis zu 360° oder geringfügig weniger, jedenfalls aber bevorzugt 300° oder mehr, betragen. Zugleich wird durch das Herausfahren der Mitnahmeelemente 29 in den Mitnahmeausformungen 30 in vertikaler Richtung Raum freigegeben für bevorzugt auch in den Mitnahmeausformungen 30 geführte Eingriffsbereiche 32 des Widerlagers 16. Das Widerlager 16 fährt nach oben und schließt bei abgenommener Verschlusskappe die Mitnehmerausformungen 30.

In einem mit der Verschlusskappe 2 abgedeckten und verschlossenen Zustand, wie er etwa Figur 1 entspricht, ist der Hohlraum 25 bzw. eine diesbezügliche fußseitige Mündung des Austragskanals 13 nicht in Überdeckung zu der Dosierkammer 19. Bevorzugt ist die Dosierkammer 19 in diesem Zustand zum Inneren der Vorratskammer 12 hin vollständig freiliegend, vgl. Figur 5. Hierdurch kann sich insbesondere schon eine gewisse Füllung der Dosierkammer 19 aus der Vorratskammer 12 ergeben haben bei einer zuvor, beim Aufschrauben der Verschlusskappe auch ausgelösten Verdrehung des Austragskanals 13 und/oder unmittelbar durch Schwerkraft aus der Vorratskammer 12.

Nach einem erfolgten Abschrauben ist zwar das Widerlager 16, wie aus Figur 6 ersichtlich, hochgefahren, befindet sich aber die Dosierkammer 19 noch in der Geschlossenstellung der Figur 5 und auch die Vorratskammer 12 noch in der Stellung der Figur 5. Wenn ein Benutzer an dem Mundstück 4 dann einen Saug-Unterdruck aufbringt, wird der Austragskanal 13 zusammen mit der Dosierkammer 12 angehoben in die in Figur 6 dargestellte Stellung. Entsprechend den Pfeilen P kann durch Öffnungen 33 in dem Rumpfteil 3 Luft in den Zwischenraum zwischen der Dosierkammer 12 und den Rumpfteil 3 gesaugt werden und von unten in die Dosierkammer 19 eintreten. Die Dosierkammer 19 entleert sich dadurch in den Austragskanal 13, und der Inhalt der Dosierkammer wird, ggf. nach Durchsetzen der Verwirbelungsmittel 14, von dem Benutzer eingesaugt.

Der Austragskanal 13 ist relativ zu dem Mundstück 4 somit aus einer ersten Stellung, in der das Ende des Austragskanals 13 einer Mündungsebene M des Mundstücks 4 näher ist, vgl. Figur 6, in eine zweite Stellung, vgl. Figur 5, in der das Ende des Austragskanals der Mündungsebene ferner ist, und umgekehrt, bewegbar. Bevorzugt und beim Ausführungsbeispiel ist der Austragskanal 12 zusammen mit der Vorratskammer 12 in dieser Hinsicht bewegbar.

Die hier auch beschriebenen Mitnahmeelemente 29 an der Verschlusskappe 2 bilden in dieser Hinsicht jedenfalls einen oder mehrere Einwirkungsabschnitte aus, um die Bewegung des Austragskanals 13 und/oder der Vorratskammer 12 zwischen der ersten und zweiten Stellung zu bewirken.

Durch das Widerlager 16 und die Feder 15 wird bei aufgeschraubter Verschlusskappe 2, siehe etwa Figuren 4 und 5, dafür gesorgt, dass die Dosierkammer 19 wieder über den Stopfen 28 gefahren ist und somit von unten verschlossen ist. Das Widerlager 16 drückt die Dosierkammer 19 - und hierbei bevorzugt auch zugleich den Austragskanal 13 - nach unten.

Die Dosierkammer 12 ist weiter oberseitig mit einer umlaufenden Dichtung 34 gegen eine Innenfläche des Rumpfteils 3 abgedichtet. Diese Dichtung bewegt sich bei einem Ab- bzw. Aufschrauben der Kappe 2 entsprechend in Vertikalrichtung entlang einer Innenfläche des Rumpfteils 3.

Der Austragskanal 13 ist bodenseitig der Vorratskammer 12 über einen Lagerzapfen 35 in dem Vorratskammer-Boden 22 drehgelagert. Bevorzugt und beim Ausführungsbeispiel ist die Ausgestaltung derart, dass eine durch den Lagerzapfen 35 gehende Drehachse zufolge der genannten Abknickung und der Ausbildung des Fußes in vertikaler Verlängerung einen Teil der freien Vorratskammer durchsetzt.

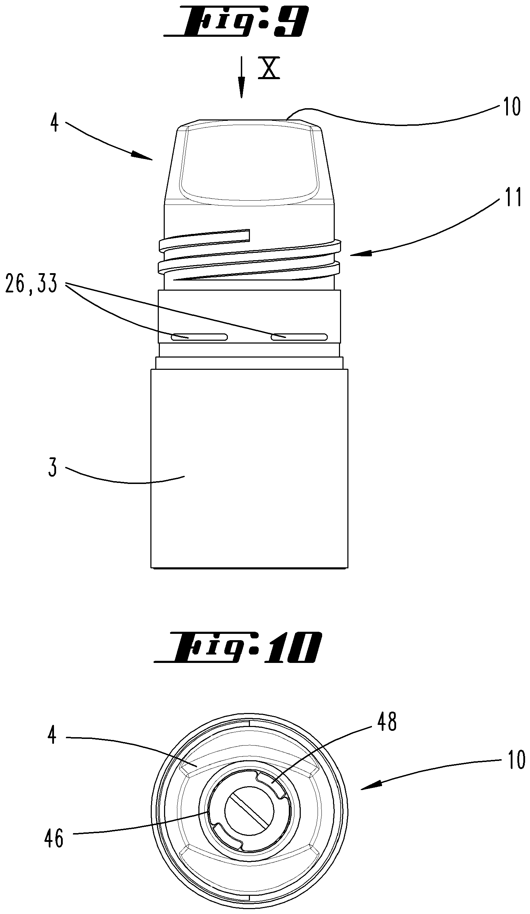

Wenn nach erfolgter Inhalation von dem Nutzer die Verschlusskappe 2 wieder aufgesetzt wird, treffen die Mitnahmeelemente 29, die beispielsweise als gegenüberliegende, in Umfangsrichtung eine Krümmung aufweisende Rippenelemente gebildet sein können, auf eine Innenfläche 46, vgl. Figur 10, die den oberen ausgefahrenen Bereich des Austragskanals 13 innerhalb einer bevorzugt zylindrischen Innenfläche 47 des Mundstücks 4 kreisringförmig verlaufend gebildet ist. Teilweise, bevorzugt an zwei gegenüberliegenden Bereichen, ist diese Fläche 46 in diesem Zustand des Inhalators durch die Stirnfläche 48 der Widerlager 16 gebildet. Die Stirnflächen 48 können in weiterer Einzelheit an parallel zu der Drehachse A verlaufenden, nach oben in Richtung der Mündung vorstehenden Eingriffsbereichen 32 des Widerlagers 16 ausgebildet sein.

Die Verschlusskappe 2 kann beim Aufsetzen nur in einen Gewindeeingriff zu dem Außengewinde 11 gebracht werden, wenn die Mitnahmeelemente 29 zuvor in einer entsprechenden Winkelausrichtung auf die genannten Stirnflächen 48 des Widerlagers 16 getroffen sind und dieses Widerlager 16 gegen die Kraft der Feder 15 um einen ersten Betrag nach unten gedrückt haben.

Weiter bevorzugt ist die Kraft der Feder 15 stärker eingestellt als ein Versetzungswiderstand der Kombination aus dem Austragskanal 13 und der Vorratskammer 12 gegen eine Versetzung nach unten, also aus der Stellung der Figur 6 in die Stellung der Figur 5. Somit wird zunächst bei einem entsprechenden Drücken der Verschlusskappe 2 in Richtung der Drehachse A, auch wenn die Mitnahmeelemente 29 bereits in Ausrichtung zu den Stirnflächen 48 sind, mit einem Niederdrücken des Widerlagers 16 eine Versetzung des Austragskanals 13 mit der Vorratskammer 12 nach unten erreicht. Hierdurch kann auch vermieden werden, dass der Austragskanal 13 relativ zu der Vorratskammer 12 bewegt wird, insbesondere gedreht, bevor die Dosierkammer 19 wieder bodenseitig verschlossen ist. Gegen weiteren Widerstand im Zuge des Aufschraubens der Verschlusskappe 2 fahren dann die Mitnahmeelemente 29 in die entsprechenden Öffnungen in der Kreisringfläche 46 tiefer ein und drücken das Widerlager 16 in die Stellung gemäß Figur 4, unter Komprimierung der Feder 15, im vollständig verschraubten Zustand der Verschlusskappe 2. Bei eingefahrenen Mitnahmeelementen 29 wird durch eine Drehung der Verschlusskappe 2 zugleich ein Drehen des Austragskanals 13 relativ zu der Vorratskammer 12 bewirkt. Hierbei ist auch bevorzugt, dass das Einfahren der Mitnahmeelemente 29 in die Mitnahmeausformungen 30 erst möglich ist, wenn ein gewisser Gewindeeingriff vorhanden ist. Die Verschlusskappe 2 kann dann nicht mehr nach oben abgezogen werden.

Weiter ist bevorzugt, dass die Gewinde der Verschlusskappe 2 und das Außengewinde 11 an dem Gehäuse des Inhalators nur in einer bestimmten Drehwinkelausrichtung der Verschlusskappe 2 ineinander greifen. Somit ist bis zum vollständigen Drehverschluss der Verschlusskappe 2 eine genau definierte Verdrehung des Austragskanals 13 relativ zu der feststehenden Vorratskammer 12 erreicht, so dass es zu der gewünschten Ausrichtung des Austragskanals 13 relativ zu der Dosierkammer 19 bei vollständig aufgeschraubter Verschlusskappe 2 kommt.

An dem Vorratskammer-Bodenteil 18 ist weiter unterseitig ein Mitnehmer 36 angebracht. Dieser Mitnehmer ist Teil des Zählwerks und wirkt mit dem Antriebsrad 20 zusammen. Das Antriebsrad 20 wirkt weiter mit einem Rücklaufverhinderer zusammen, der in Einzelheit nicht dargestellt ist.

Bei einem Hochfahren der Vorratskammer 12 und insbesondere des Vorratskammer-Bodens 22 wird zufolge des Mitnehmers 36 das Antriebsrad 20 um einen Winkelbetrag mitgenommen. Das Antriebsrad 20 ist bevorzugt in Form einer Schneckenwelle, weiter bevorzugt einer eingängigen Schneckenwelle, ausgebildet, mit einer Schneckenwendelsteigung, die angepasst ist an die Beabstandung von Mitnahmevorsprüngen 37 an dem Zählring 7. Die Mitnahmevorsprünge 37 sind bezüglich des Zählrings 7 ersichtlich nach innen gerichtet. Ein jeweils dem Antriebsrad 20 zugeordneter Mitnahmevorsprung tritt etwa auf einer Höhe einer Drehachse des Antriebsrades 20 zwischen die Flanken der Schneckenwendel ein. Bei einer Drehverlagerung des Antriebsrades 20 erfolgt entsprechend eine Verlagerung des gefassten Mitnahmevorsprungs in Horizontalrichtung, d.h. quer zu der Drehachse A. Hierdurch wird das Zählrad 7 entsprechend gedreht.

Mit jedem Zuschrauben der Kappe 2 ergibt sich also eine Weiterdrehung des Zählrades 7.

Im Weiteren wird hinsichtlich der Ausgestaltung des Zählwerks auch auf die WO 2007/104698 A1, insbesondere die Figuren 13 bis 19 und die zugehörige Beschreibung verwiesen, darüber hinaus auch auf die dazu parallele US 8 261 737 B2, hinsichtlich der Figuren selber Nummerierung und der diesbezüglichen Beschreibung.

Mit dem Unterschied, dass die Zählung bei dem Gegenstand der genannten Druckschriften mit dem Niederdrücken zum Beladen der Dosierkammer erfolgt, und der Mitnehmer 36 im Ruhezustand, hier bei aufgeschraubter Kappe 2, in Überdeckung zu dem Antriebsrad 20 ist, ist eine gleiche Funktion und Ausgestaltung gegeben.

Der Inhalator 1 hat insgesamt in Erstreckung der Drehachse A eine Länge, die an die Breite einer Hand eines menschlichen Benutzers angepasst ist, beispielsweise bis hin zur zweifachen, dreifachen Breite einer Hand eines menschlichen Benutzers. Somit kann der Inhalator in dieser Erstreckung eine Länge von beispielsweise 7 bis 20 cm aufweisen.

Der Durchmesser im zylindrischen Bereich kann beispielsweise 5 bis 10 cm betragen.

Der Fuß 21 weist mit Bezug zu den Figuren 7 und 8 radial innen, etwa überdeckend in vertikaler zu dem Lagerzapfen 35 eine relativ steile Flanke 38 auf, die nach radial außen in eine Schaufelfläche 39 übergeht. Die Flanke 38 kann mit einer Vertikalen einen spitzen Winkel von 0° bis 30° einschließen, während die Schaufelfläche 39, ggf. auch unterschiedlich gewinkelt in Bezug auf ihre radiale Erstreckung, einen Winkel mit einer Vertikalen im Bereich von 20° bis 40° einschließen kann. Unabhängig von der konkret gewählten Winkelausrichtung ist immer bevorzugt, dass die Flanke 38 steiler ist als die Schaufelfläche 39. Diese Gestaltung ist ersichtlich in Drehrichtung einseitig gegeben, während gegenüberliegend nur eine durchgehende Gegenflanke 40 ausgebildet ist. Die Gegenflanke 40 ist bevorzugt ebenfalls steil ausgebildet. Sie kann auch einen Winkel mit einer der Drehachse A entsprechenden Vertikalen von 0° bis 30° einschließen.

Bevorzugt ist auch hinsichtlich des Austragskanals 13 ein Drehanschlag ausgebildet. Hierzu kann an dem Austragskanal 13 ein Anschlagvorsprung 41 ausgebildet sein und an der Vorratskammer 12, nämlich einer Vorratskammerdecke, deren vertikal durchgehende Öffnung der Austragskanal 13 durchsetzt, ein entsprechender Gegenanschlag. Somit ist jedenfalls keine vollständige 360°-Drehung ermöglicht.

**Liste der Bezugszeichen**

| | | | |
|---|---|---|---|
| 1 | Inhalator | 29 | Mitnahmeelement |
| 2 | Verschlusskappe | 30 | Mitnahmeausformung |
| 3 | Rumpfteil | 31 | Ausformung |
| 4 | Mundstück | 32 | Eingriffsbereich |
| 5 | Bodenteil | 33 | Öffnung |
| 6 | Fenster | 34 | Dichtung |
| 7 | Zählring | 35 | Lagerzapfen |
| 8 | Reservoir | 36 | Mitnehmer |
| 9 | Verbindungsteil | 37 | Mitnahmevorsprung |
| 10 | Mündungsabschnitt | 38 | Flanke |
| 11 | Außengewinde | 39 | Schaufelfläche |
| 12 | Vorratskammer | 40 | Gegenflanke |
| 13 | Austragskanal | 41 | Anschlagvorsprung |
| 14 | Verwirbelungsmittel | 42 | Teilbereich |
| 15 | Feder | 43 | Teilbereich |
| 16 | Widerlager | 44 | Teilungskante |
| 17 | Dichtung | 45 | Teilungskante |
| 18 | Vorratskammer-Bodenteil | 46 | Innenfläche/Kreisringfläche |
| 19 | Dosierkammer | 47 | Innenfläche |
| 20 | Antriebsrad | 48 | Stirnfläche |
| 21 | Fuß | | |
| 22 | Vorratskammer-Boden | A | Drehachse |
| 23 | Kammerwand | AL | Ansaugluft |
| 24 | Lagervorsprung | α | Winkel |
| 25 | Höhlung | | |
| 26 | Durchlassöffnung | E | Ebene |
| 27 | Luftkanal | L | Längsachse |
| 28 | Stopfen | L₁ | Längsachse |
| M | Mündungsebene | | |
| P | Pfeil | | |
| T₁ | Teilstrom | | |
| T₂ | Teilstrom | | |
| T₃ | Teilstrom | | |
| T₄ | Teilstrom | | |
| V | Vertikale | | |

## Patentansprüche

1. Handbetätigbarer Inhalator (1) zur Ausbringung einer pulverförmigen Substanz, insbesondere einer pharmazeutischen Substanz, mit einer Vorratskammer, einem Austragskanal (13) und einer Dosierkammer (19), wobei der Austragskanal in die Vorratskammer (12) hineinragt und die Dosierkammer (19) und der Austragskanal (13) relativ zueinander bewegbar sind, wobei der Austragskanal (13) um eine Drehachse (A) drehbar ist und die Dosierkammer (19) im Zug einer Drehung des Austragskanals (13) füllbar ist, **dadurch gekennzeichnet, dass** die Dosierkammer (19) auf ihrer dem Austragskanal (13) gegenüber liegenden Seite eine durch einen Stopfen (28) verschließbare Öffnung aufweist, und dass die Dosierkammer (19) von dem Stopfen (28) abhebbar ist.

2. Handbetätigbarer Inhalator (1) nach Anspruch 1, **dadurch gekennzeichnet, dass** die Vorratskammer (12) zusammen mit dem Austragskanal (13) für eine Versetzung der Dosierkammer (19) von einem geschlossenen in einen geöffneten Zustand und umgekehrt relativ zu dem Mundstück (4) versetzbar ist.

3. Handbetätigbarer Inhalator (1) nach einem der vorhergehenden Ansprüche, wobei in dem Austragskanal (13) ein Verwirbelungsmittel (14) angeordnet ist, das eine wendelförmig verlaufende Wandung aufweist und bei einem Austragen von mit der Substanz versehener Luft zu durchströmen ist, **dadurch gekennzeichnet, dass** die Wandung in einem in einer Durchströmungsrichtung gegebenen Eintrittsbereich die durchströmende Luft in danach nebeneinander geführte Teilströme (T₁, T₂) aufteilt und in Durchströmungsrichtung nachfolgend die ersten Teilströme (T₁, T₂) in einem Unterbrechungsbereich in zweite, danach auch nebeneinander geführte Teilströme (T₃, T₄) aufgeteilt werden, wobei ein zweiter Teilstrom (T₃, T₄) jeweils auf einer Zusammenführung von Anteilen der der beiden ersten Teilströme (T₁, T₂) beruht, dass das Verwirbelungsmittel (14) aus in einer axialen Richtung hintereinander angeordneten Teilbereichen (42, 43) besteht, die in einer Ebene (E-E), die senkrecht zu einer Längsachse (L1) des Verwirbelungsmittels (14) verläuft, aus einem diese Teilbereiche (42, 43) enthaltenden kontinuierlichen Wendeteil gedanklich getrennt und dann winkelmäßig versetzt wieder zusammengesetzt sind.

4. Handbetätigbarer Inhalator (1) nach einem der vorhergehenden Ansprüche, **dadurch gekennzeichnet, dass** das Mundstück (4) eine Mündungsebene aufweist, dass eine Verschlusskappe (2) zum Überfangen des Mundstücks (4) sowie jedenfalls eines dem Mundstück (4) zugeordneten Endes des Austragskanals (13) vorgesehen ist, dass der Austragskanal (13) relativ zu dem Mundstück (4) aus einer ersten Stellung, in der das Ende des Austragskanals (13) der Mündungsebene näher ist, in eine zweite Stellung, in der das Ende des Austragskanals der Mündungsebene ferner ist, und umgekehrt, bewegbar ist, und dass an der Verschlusskappe (2) ein Einwirkungsabschnitt vorgesehen ist, zur Bewirkung jedenfalls der Bewegung des Austragskanals (13) und/oder der Vorratskammer (12) aus der ersten in die zweite Stellung.

5. Inhalator nach einem der vorhergehenden Ansprüche, **dadurch gekennzeichnet, dass** die Drehachse (A) zumindest teilweise entlang einer durch den Austragskanal (13) vorgegebenen Transportrichtung von in dem Austragskanal transportierter Substanz verläuft.

6. Inhalator nach Anspruch 5, **dadurch gekennzeichnet, dass** die Dosierkammer (19) zu der Drehachse des Austragskanals (13) exzentrisch angeordnet ist.

7. Inhalator nach einem der vorhergehenden Ansprüche , **dadurch gekennzeichnet, dass** die Dosierkammer (19) von dem Austragskanal (13) gesondert ausgebildet ist.

8. Inhalator nach einem der vorhergehenden Ansprüche , **dadurch gekennzeichnet, dass** die Dosierkammer (19) bodenseitig der Vorratskammer (12) gelegen ist.

9. Inhalator nach einem der vorhergehenden Ansprüche, **dadurch gekennzeichnet, dass** der Austragskanal (13) einen Fuß aufweist, der im Zuge einer Drehung des Austragskanals (13) die Dosierkammer (19) zur Füllung freigibt.

10. Inhalator nach einem der vorhergehenden Ansprüche, **dadurch gekennzeichnet, dass** eine Unterseite des Austragskanals (13) ebenengleich zu einem Boden (22) der Vorratskammer außerhalb der Dosierkammer (19) verläuft.

11. Inhalator nach einem der vorhergehenden Ansprüche, **dadurch gekennzeichnet, dass** der Austragskanal (13) hinsichtlich eines darin gebildeten Strömungsweges einen abgewinkelten Verlauf aufweist.

12. Inhalator nach einem der vorhergehenden Ansprüche, **dadurch gekennzeichnet, dass** die Dosierkammer (19) zusammen mit dem Austragskanal (13) bewegbar ist.

## Claims

1. Hand-operated inhaler (1) for dispensing a powdery substance, in particular a pharmaceutical substance, comprising a storage chamber, a discharge channel (13) and a dosing chamber (19), the discharge channel protruding into the storage chamber (12) and the dosing chamber (19) and the discharge channel (13) being movable relative to one another, the discharge channel (13) being rotatable about an axis of rotation (A) and it being possible to fill the dosing chamber (19) in the course of a rotation of the discharge channel (13), **characterised in that** the dosing chamber (19) comprises, on the side thereof opposite the discharge channel (13), an opening which can be closed by a stopper (28), and **in that** the dosing chamber (19) can be lifted up from the stopper (28).

2. Hand-operated inhaler (1) according to claim 1, **characterised in that** the storage chamber (12), together with the discharge channel (13), can be displaced relative to the mouthpiece (4) for displacement of the dosing chamber (19) from a closed to an open state and vice versa.

3. Hand-operated inhaler (1) according to either of the preceding claims, a swirling means (14) being arranged in the discharge channel (13), which means has a helically extending wall and through which means air provided with the substance can flow when discharging, **characterised in that** the wall in an inlet region provided in a direction of flow divides the air flowing through into partial streams (T₁, T₂) that are subsequently guided adjacently and, in the direction of flow, the first partial streams (T₁, T₂) are subsequently divided in an interruption region into second partial streams (T₃, T₄) that are also subsequently guided adjacently, a second partial stream (T₃, T₄) being based in each case on a convergence of portions of the two first partial streams (T₁, T₂), **in that** the swirling means (14) consists of partial regions (42, 43) which are arranged one after the other in an axial direction and, in a plane (E-E) which extends perpendicularly to a longitudinal axis (L1) of the swirling means (14), are theoretically separated out of a continuous helical part containing these partial regions (42, 43) and then brought together again so as to be offset at an angle.

4. Hand-operated inhaler (1) according to any of the preceding claims, **characterised in that** the mouthpiece (4) has an opening plane, **in that** a closure cap (2) is provided for covering the mouthpiece (4) and in any case an end of the discharge channel (13) associated with the mouthpiece (4), **in that** the discharge channel (13) can be moved relative to the mouthpiece (4) from a first position, in which the end of the discharge channel (13) is closer to the opening plane, to a second position, in which the end of the discharge channel is further away from the opening plane, and vice versa, and **in that** an influencing portion is provided on the closure cap (2) for causing in any case the movement of the discharge channel (13) and/or the storage chamber (12) from the first to the second position.

5. Inhaler according to any of the preceding claims, **characterised in that** the axis of rotation (A) extends at least in part in a transport direction, predefined by the discharge channel (13), for a substance transported in the discharge channel.

6. Inhaler according to claim 5, **characterised in that** the dosing chamber (19) is arranged eccentrically to the axis of rotation of the discharge channel (13).

7. Inhaler according to any of the preceding claims, **characterised in that** the dosing chamber (19) is formed separately from the discharge channel (13).

8. Inhaler according to any of the preceding claims, **characterised in that** the dosing chamber (19) is located at the bottom of the storage chamber (12).

9. Inhaler according to any of the preceding claims, **characterised in that** the discharge channel (13) has a base which releases the dosing chamber (19) for filling in the course of a rotation of the discharge channel (13).

10. Inhaler according to any of the preceding claims, **characterised in that** an underside of the discharge channel (13) extends in the same plane as a bottom (22) of the storage chamber outside the dosing chamber (19).

11. Inhaler according to any of the preceding claims, **characterised in that** the discharge channel (13) has an angled course with respect to a flow path formed therein.

12. Inhaler according to any of the preceding claims, **characterised in that** the dosing chamber (19) is movable together with the discharge channel (13).

## Revendications

1. Inhalateur à actionnement manuel (1) pour distribuer une substance pulvérulente, en particulier une substance pharmaceutique, comprenant une chambre de stockage, un canal de décharge (13) et une chambre de dosage (19), dans lequel le canal de décharge fait saillie dans la chambre de stockage (12) et la chambre de dosage (19) et le canal de décharge (13) sont mobiles l'un par rapport à l'autre, dans lequel le canal de décharge (13) peut tourner autour d'un axe de rotation (A) et la chambre de dosage (19) peut être remplie au cours d'une rotation du canal de décharge (13), **caractérisé en ce que** la chambre de dosage (19) présente, sur son côté opposé au canal de décharge (13), une ouverture qui peut être obturée par un bouchon (28), et que la chambre de dosage (19) peut être dégagée du bouchon (28).

2. Inhalateur à actionnement manuel (1) selon la revendication 1, **caractérisé en ce que** la chambre de stockage (12) ensemble avec le canal de décharge (13) sont déplaçables par rapport à l'embout (4) pour réaliser un déplacement de la chambre de dosage (19) d'un état fermé à un état ouvert et vice versa.

3. Inhalateur à actionnement manuel (1) selon l'une des revendications précédentes, dans lequel un moyen de génération de turbulences (14) est agencé dans le canal de décharge (13), lequel moyen de génération de turbulences (14) comprend une paroi s'étendant en hélice par lequel doit passer l'écoulement lors d'une décharge d'air pourvu de la substance, **caractérisé en ce que** dans une région d'entrée définie dans un sens d'écoulement, la paroi divise l'air en flux partiels (T1, T2) guidés ensuite côte à côte, puis, en aval dans le sens d'écoulement, divise les premiers flux partiels (T1, T2) dans une zone d'interruption en deuxièmes flux partiels (T3, T4) qui sont également guidés ensuite côte à côte, dans lequel un deuxième flux partiel (T3, T4) est basé à chaque fois sur la réunion de parties des deux premiers flux partiels (T1, T2), **en ce que** le moyen de turbulence (14) est constitué de régions partielles (42, 43) agencées successivement dans une direction axiale qui, dans un plan (E-E) s'étendant perpendiculairement à un axe longitudinal (L1) du moyen de génération de turbulences (14), sont séparées mentalement d'une partie tournante continue qui contient ces régions partielles (42, 43) et sont ensuite réassemblées avec un décalage angulaire.

4. Inhalateur à actionnement manuel (1) selon l'une des revendications précédentes, **caractérisé en ce que** l'embout (4) présente un plan d'embouchure, **en ce que** sont prévus un capuchon de fermeture (2) pour recouvrir l'embout (4) et au moins une extrémité du canal de décharge (13) qui est associée à l'embout (4), **en ce que** le canal de décharge (13) est déplaçable par rapport à l'embout (4) depuis une première position dans laquelle l'extrémité du canal de décharge (13) est plus proche du plan d'embouchure, dans une deuxième position dans laquelle l'extrémité du canal de décharge est plus éloignée du plan d'embouchure, et vice versa, et **en ce qu'**une partie d'actionnement est prévue sur le bouchon de fermeture (2) pour provoquer tout au moins le mouvement du canal de décharge (13) et/ou de la chambre de stockage (12) depuis la première position vers la deuxième position.

5. Inhalateur selon l'une des revendications précédentes, **caractérisé en ce que** l'axe de rotation (A) s'étend au moins partiellement le long d'une direction de transport de substance transportée dans le canal de décharge qui est prédéterminée par le canal de décharge (13).

6. Inhalateur selon la revendication 5, **caractérisé en ce que** la chambre de dosage (19) est agencée de manière excentrique par rapport à l'axe de rotation du canal de décharge (13).

7. Inhalateur selon l'une des revendications précédentes, **caractérisé en ce que** la chambre de dosage (19) est formée séparée du canal de décharge (13).

8. Inhalateur selon l'une des revendications précédentes, **caractérisé en ce que** la chambre de dosage (19) est située du côté inférieur de la chambre de stockage (12).

9. Inhalateur selon l'une des revendications précédentes, **caractérisé en ce que** le canal de décharge (13) présente un pied qui, au cours d'une rotation du canal de décharge (13), libère la chambre de dosage (19) pour remplissage.

10. Inhalateur selon l'une des revendications précédentes, **caractérisé en ce qu'**un côté inférieur du canal d'évacuation (13) s'étend en dehors de la chambre de dosage (19) au même niveau qu'un fond (22) de la chambre de stockage.

11. Inhalateur selon l'une des revendications précédentes, **caractérisé en ce que** le canal de décharge (13) présente un cheminement anguleux par rapport à un trajet d'écoulement formé dans celui-ci .

12. Inhalateur selon l'une des revendications précédentes, **caractérisé en ce que** la chambre de dosage (19) est mobile ensemble avec le canal de décharge (13).
